# EUROPÄISCHE PATENTSCHRIFT

(11) **EP 2 964 073 B1**
(45) Veröffentlichungstag und Bekanntmachung des Hinweises auf die Patenterteilung: **30.05.2018**
(21) Anmeldenummer: 14702836.9
(22) Anmeldetag: 04.02.2014
(51) Int. Cl.: A61B 1/247, A61C 19/04, A61C 1/08

(54) **SPIEGEL, INSBESONDERE MEDIZINISCHER SPIEGEL, UND VORRICHTUNG, INSBESONDERE ZUR WURZELKANALBEHANDLUNG**
MIRROR, IN PARTICULAR MEDICAL MIRROR, AND DEVICE, IN PARTICULAR FOR ROOT CANAL TREATMENT
MIROIR, EN PARTICULIER MIROIR MÉDICAL, ET DISPOSITIF, EN PARTICULIER POUR L'ENDODONTIE

(30) Priorität: 07.03.2013 DE 202013002440 U; 15.03.2013 DE 102013004876
(43) Veröffentlichungstag der Anmeldung: 13.01.2016
(73) Patentinhaber: Winkelmann, Wolfgang, 72076 Tübingen (DE)
(72) Erfinder: Winkelmann, Wolfgang, 72076 Tübingen (DE)
(74) Vertreter: Wohlfahrt, Jan Günther
(86) Internationale Anmeldenummer: PCT/EP2014/052094
(87) Internationale Veröffentlichungsnummer: WO 2014/135320

(56) Entgegenhaltungen:
- DE-A1-102005 034 897
- DE-C- 420 594
- US-A- 1 086 887
- US-A- 2 679 103
- US-A- 3 300 859
- US-A1- 2011 058 716

## Beschreibung

Die vorliegende Erfindung betrifft neuartige Spiegel, insbesondere medizinische Spiegel, bevorzugt Dentalspiegel, auch Mundspiegel genannt, die ein Loch aufweisen, durch das zahnmedizinische Instrumente, insbesondere Wurzelkanalinstrumente oder Guttapercha-Stifte hindurch gesteckt werden können, sowie Vorrichtungen, die einen erfindungsgemäßen Dentalspiegel aufweisen.

Spiegel werden bei Operationen, insbesondere auch bei endodontologischen und endologischen Operationen, häufig eingesetzt um die zu behandelnden Körperbereiche, beispielsweise Zähne, und die Operationsschritte gut zu sehen. Dabei sollte während der Durchführung eines Operationsschrittes die entsprechende Stelle für den behandelnden Arzt oder Zahnarzt gleichzeitig über den Spiegel einsehbar sein. Entsprechende medizinische Spiegel sind bekannt.

Auch Dentalspiegel sind hinlänglich bekannt. Sie umfassen typischer Weise mindestens eine runde Spiegelfläche, die planar oder konkav sein kann. Die Spiegelfläche befindet sich auf einem Spiegelgrundkörper, an dem sich meist ein Griffstück befindet. Typische Durchmesser der Spiegelfläche von Dentalspiegeln sind 21 mm (Größe 4) über 24 mm (Größe 5) bis 30 mm (Größe 8).

Bei Operationen an schwer einsehbaren Köperstellen, wie zum Beispiel den Backenzähnen, ist es kaum möglich die für die Operation notwendigen Instrumente und gleichzeitig einen medizinischen Spiegel, insbesondere Dentalspiegel, an der Operationsstelle einzusetzen, wobei der Spiegel gleichzeitig die entsprechende Stelle für den behandelnden Arzt sichtbar machen soll. Zwei Dentalspiegel sind aus dem Stand der Technik US 3,300,859 und US 2,679,103 bekannt. Das der vorliegenden Erfindung zugrunde liegende technische Problem besteht daher darin, einen Dentalspiegel bereitzustellen, der bei Operationen und Behandlungen an schwer zugänglichen Körperstellen verwendet werden kann, ohne dass er die Verwendung der Operationsinstrumente behindert aber gleichzeitig dem behandelnden Arzt den Operationsschritt gut sichtbar macht.

Die vorliegende Erfindung löst das ihr zugrunde liegende technische Problem durch die Bereitstellung eines Spiegels nach Anspruch 1 und einer Vorrichtung, umfassend den erfindungsgemäßen Spiegel, nach Anspruch 11. Die vorliegende Erfindung löst das ihr zugrunde liegende technische Problem insbesondere durch die Bereitstellung eines Spiegels, aufweisend eine Spiegelfläche, wobei die Spiegelfläche ein Loch aufweist. Das Loch reicht also durch den Spiegelgrundkörper, der die Spiegelfläche trägt, hindurch. Der Spiegel ist ein Dentalspiegel, auch Mundspiegel genannt.

Bevorzugt wird die Spiegelfläche von einem Spiegelgrundkörper in bekannter Art und Weise getragen, der Spiegelgrundkörper ist also zumindest auf einer Seite bevorzugt mit einer Spiegelfläche beschichtet.

Das Loch kann in vorteilhafter Weise dazu genutzt werden, dass ein Operationsinstrument, insbesondere ein längliches Operationsinstrument, wie zum Beispiel oft verwendete Dentalinstrumente oder ein Guttapercha-Stift durch das Loch hindurchgeschoben werden können und somit insbesondere während der Behandlung durch das Loch hindurchreichen. Der Spiegel umgibt somit das Operationsinstrument und spiegelt die Operationsstelle wider, so dass sie vom Arzt gut gesehen werden kann. Dabei kann das Operationsinstrument uneingeschränkt verwendet werden und der Spiegel so entlang der Achse des Operationsinstruments so gekippt werden, dass der Arzt ein möglichst gutes Bild erhält.

In einer bevorzugten Ausführungsform weist das Loch einen Durchmesser von mindestens 1 mm und höchstens 11 mm auf. In einer bevorzugten Ausführungsform weist das Loch einen Durchmesser von mindestens 1 mm auf. In einer bevorzugten Ausführungsform weist das Loch einen Durchmesser von mindestens 2 mm auf. In einer bevorzugten Ausführungsform weist das Loch einen Durchmesser von höchstens 7 mm auf. In einer bevorzugten Ausführungsform weist das Loch einen Durchmesser von höchstens 5 mm auf. In einer bevorzugten Ausführungsform weist das Loch einen Durchmesser von mindestens 1 mm und höchstens 3 mm auf.

Das Loch muss einen Durchmesser haben, der mindestens so groß ist wie der Durchmesser des Operationsinstruments, das durch das loch hindurchreichen soll. Das Loch hat bevorzugt einen Durchmesser, der etwas größer ist als der Durchmesser des Operationsinstruments, das durch das Loch hindurchreichen soll, so dass der Spiegel dadurch entlang der Achse des Operationsinstruments gekippt werden kann, so dass das projizierte Bild verstellt werden kann und der Arzt die zu behandelnde Stelle durch das Spiegelbild besonders gut sieht.

In einer bevorzugten Ausführungsform hat die Spiegelfläche einen größten Durchmesser von höchstens 5 cm. In einer bevorzugten Ausführungsform hat die Spiegelfläche einen größten Durchmesser von höchstens 3,5 cm. In einer bevorzugten Ausführungsform hat die Spiegelfläche einen größten Durchmesser von höchstens 2,5 cm. In einer bevorzugten Ausführungsform hat die Spiegelfläche einen Durchmesser von mindestens 0,5 cm.

In einer bevorzugten Ausführungsform befindet das Loch etwa in der Mitte der Spiegelfläche. In einer bevorzugten Ausführungsform ist das Loch in der Mitte der Spiegelfläche zentriert. Das Loch weist auf der Rückseite des die Spiegelfläche tragenden Spiegelgrundkörpers abgeflachte oder abgerundete Kanten auf. Dies verbessert in vorteilhafter Weise die Kippmöglichkeit des Spiegels entlang der Achse des durchreichenden Operationsinstruments.

In einer bevorzugten Ausführungsform ist die Spiegelfläche rund. In einer bevorzugten Ausführungsform ist die Grundform der Spiegelfläche rund. Die Spiegelfläche kann natürlich aber auch jegliche andere Form haben, zum Beispiel kann die Spiegelfläche oval, dreieckig, quadratisch, viereckig oder vieleckig sein. Die Spiegelfläche weist zwei konkave Ausnehmungen auf. Die erste Ausnehmung oder Einkerbung kann in vorteilhafter Weise dazu dienen, dass insbesondere ein Dentalspiegel möglichst nah an das Arbeitsfeld zum Beispiel an die Wurzelkanaleingänge eines Zahns herangeführt werden kann. Die zweite Ausnehmung oder Einkerbung kann in vorteilhafter Weise dazu dienen, dass das Operationsinstrument, das durch das Loch hindurchreicht, zum Beispiel der Bohrkopf eines Winkelstücks, einen möglichst großen Freiheitsgrad hat, insbesondere wenn der Spiegel entlang der Instrumentenachse abgeknickt ist.

In einer bevorzugten Ausführungsform weist der Spiegel, insbesondere der Dentalspiegel ein Griffstück auf. Alternativ kann auch vorgesehen sein, dass kein Griffstück vorhanden ist, beispielsweise wenn der Spiegel in einer alternativen Ausführungsform der erfindungsgemäßen Vorrichtung verwendet wird, bei der der Spiegel an einem Operationsinstrument, insbesondere einem Winkelstück, befestigt ist.

In einer bevorzugten Ausführungsform steht die Mitte der mindestens einen konkaven Ausnehmung in einem Winkel von 0° bis 90°, insbesondere von etwa 45°, zu der Längsachse eines Griffstücks. Das Loch ist über eine Spalte oder einen Schlitz mit der Spiegelflächenaußenkante verbunden. Dies ermöglicht ein einfaches Einführen oder Entfernen eines Operationsinstruments. Bevorzugt ist die Breite der Spalte oder des Schlitzes geringer als der Durchmesser des Lochs aber größer als der Durchmesser des Operationsinstruments.

In einer bevorzugten Ausführungsform mündet die Spalte in eine konkave Ausnehmung der Spiegelfläche.

In einer bevorzugten Ausführungsform ist die Spiegelfläche eine planare Spiegelfläche. In einer alternativen Ausführungsform ist die Spiegelfläche konkav. In einer alternativen Ausführungsform ist die Spiegelfläche eine vergrößernde Spiegelfläche.

In einer bevorzugten Ausführungsform weist der Dentalspiegel am Griffstück einen isolierten elektrischen Anschluss auf, der über eine isolierte elektrische Leitung mit einer aus einem elektrisch leitenden Material hergestellte Kontaktstelle verbunden ist, wobei sich die Kontaktstelle an der Innenwandfläche des Lochs oder auf der Rückseite der Spiegelfläche befindet. So ist in vorteilhafter Weise eine elektronische Messung der Wurzelkanaltiefe möglich, ohne dass eine Elektrode störend an das Wurzelkanalinstrument herangebracht werden muss.

Die vorliegende Erfindung löst das ihr zugrunde liegende technische Problem auch durch eine Vorrichtung zur Behandlung von Zähnen, umfassend einen erfindungsgemäßen Spiegel, insbesondere erfindungsgemäßen Dentalspiegel, ein Winkelstück und ein mit dem Winkelstück verbundenes Dentalinstrument, wobei das Dentalinstrument durch das Loch des Dentalspiegels hindurchreicht.

In einer bevorzugten Ausführungsform dient die Vorrichtung zur endodontologischen Behandlung von Zähnen, und umfasst einen erfindungsgemäßen Dentalspiegel, ein Winkelstück und ein mit dem Winkelstück verbundenes Wurzelkanalinstrument, wobei das Wurzelkanalinstrument durch das Loch des Dentalspiegels hindurchreicht.

In einer bevorzugten Ausführungsform ist das Wurzelkanalinstrument eine Nervennadel, eine Feile, ein Fräser, ein Bohrer oder ein Reamer ist.

In einer bevorzugten Ausführungsform ist der Spiegel dem Dentalinstrument, insbesondere dem Wurzelkanalinstrument, und dem Winkelstück zugeordnet. In einer alternativen Ausführungsform ist der Spiegel mit dem Winkelstück verbunden.

In einer bevorzugten Ausführungsform kommt das Dentalinstrument, insbesondere Wurzelkanalinstrument mit der Innenfläche des Lochs und/oder der Rückseite der Spiegelfläche in Kontakt. Dadurch ist insbesondere in der Ausführungsform des Dentalspiegels, bei der der Dentalspiegel am Griffstück einen isolierten elektrischen Anschluss aufweist, der über eine isolierte elektrische Leitung mit einer aus einem elektrisch leitenden Material hergestellte Kontaktstelle verbunden ist, wobei sich die Kontaktstelle an der Innenwandfläche des Lochs oder auf der Rückseite der Spiegelfläche befindet, eine elektronische Messung der Wurzelkanaltiefe möglich.

Die vorliegende Erfindung löst das ihr zugrunde liegende technische Problem auch durch einen erfindungsgemäßen Spiegel zur Verwendung bei einer medizinischen Operation. Bevorzugt wird dabei der Spiegel so verwendet, dass ein Operationsinstrument durch das Loch des Spiegels hindurchreicht.

Die vorliegende Erfindung löst das ihr zugrunde liegende technische Problem auch durch einen erfindungsgemäßen Dentalspiegel zur Verwendung zur Behandlung von Zähnen. Bevorzugt wird dabei der Dentalspiegel so verwendet, dass ein Dentalinstrument durch das Loch des Dentalspiegels hindurchreicht.

Die vorliegende Erfindung löst das ihr zugrunde liegende technische Problem auch durch die Verwendung eines erfindungsgemäßen Spiegels bei einer medizinischen Operation. Bevorzugt wird dabei der Spiegel so verwendet, dass ein Operationsinstrument durch das Loch des Spiegels hindurchreicht.

Die vorliegende Erfindung löst das ihr zugrunde liegende technische Problem auch durch die Verwendung eines erfindungsgemäßen Dentalspiegels zur Behandlung von Zähnen. Bevorzugt wird dabei der Dentalspiegel so verwendet, dass ein Dentalinstrument durch das Loch des Dentalspiegels hindurchreicht.

Die vorliegende Erfindung löst das ihr zugrunde liegende technische Problem auch durch Verfahren zur medizinischen Behandlung, enthaltend die Schritte a) Hindurchstecken eines Operationsinstruments durch das Loch des erfindungsgemäßen Spiegels und b) Verwenden des Operationsinstruments an einer zu behandelnden Stelle und gleichzeitiges Betrachten der zu behandelnden Stelle über den Spiegel.

Die vorliegende Erfindung löst das ihr zugrunde liegende technische Problem auch durch Verfahren zur zahnmedizinischen Behandlung, enthaltend die Schritte a) Hindurchstecken eines Dentalinstrument oder Guttapercha-Stifts durch das Loch des erfindungsgemäßen Dentalspiegels und b) Verwenden des Dentalinstruments an einem zu behandelnden Zahn und gleichzeitiges Betrachten des zu behandelnden Zahns über den Dentalspiegel.

Die vorliegende Erfindung wird anhand der nachfolgenden Figuren beispielhaft näher erläutert ohne dass die Figuren einschränkend zu verstehen wären.

Die Figuren zeigen:
- Figur 1: einen erfindungsgemäßen Dentalspiegel mit einem Griffstück;
- Figur 2A: einen erfindungsgemäßen Dentalspiegel mit einer Spalte;
- Figur 2B: erfindungsgemäße Dentalspiegel mit einer Spalte, einer Ausnehmung und einem Griffstück;
- Figur 2C: einen erfindungsgemäßen Dentalspiegel mit zwei konkaven Ausnehmungen und einem Griffstück;
- Figur 3A: einen erfindungsgemäßen Dentalspiegel mit einer konkaven Ausnehmung und einem Griffstück vor der Behandlung eines Zahns;
- Figur 3B: einen erfindungsgemäßen Dentalspiegel mit einer konkaven Ausnehmung und einem Griffstück bei der Behandlung eines Zahns mit einem Dentalinstrument;
- Figur 4A: Herkömmliche Behandlung eines Zahns mit einem Dentalspiegel aus dem Stand der Technik und herkömmlicher elektronscher Wurzelkanaltiefenmessung;
- Figur 4B: Erfindungsgemäße Behandlung und elektronscher Wurzelkanaltiefenmessung eines Zahns mit einem erfindungsgemäßen Dentalspiegel;
- Figur 5: eine erfindungsgemäße Vorrichtung mit einem erfindungsgemäßen Spiegel und einem durch den Spiegel hindurchreichenden Wurzelkanalinstrument, das mit einem Winkelstück verbunden ist.

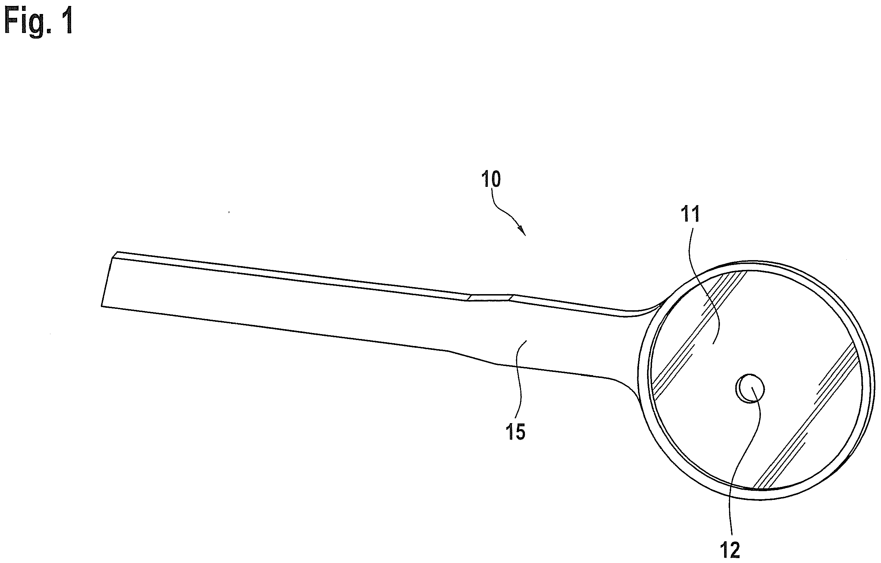

Figur 1 zeigt einen erfindungsgemäßen Dentalspiegel (10). Dieser hat eine aus dem Stand der Technik bekannte Spiegelfläche (11) auf einem Spiegelgrundkörper und ein aus dem Stand der Technik bekanntes Griffstück (15). Typischerweise ist die Spiegelfläche eines Dentalspiegels rund. Der erfindungsgemäße Spiegel zeichnet sich dadurch aus, dass die Spiegelfläche (11) ein Loch (12) aufweist, das bevorzugt etwa mittig in der Spiegelfläche positioniert ist. Dieses Loch kann in vorteilhafter Weise beispielsweise bei einer Wurzelkanalbehandlung, insbesondere bei Backenzähnen und ganz besonders bei oberen Backenzähnen dazu verwendet werden, ein zur Wurzelkanalbehandlung benötigtes Instrument, beispielsweise eine Nadel, eine Fräse, eine Feile oder einen Guttapercha-Stift hindurchzuführen, so dass das Instrument zur Behandlung eingesetzt werden kann und gleichzeitig der Spiegel so positioniert werden kann, dass die Behandlungsstelle für den Zahnarzt gut sichtbar. Die Größe des Lochs (12) ist daher bevorzugt etwas größer als zur Wurzelkanalbehandlung verwendete Instrumente, so dass diese hindurch passen und der Spiegel gegenüber der Achse der Instrumente verdreht werden kann, so dass die Behandlungsstelle gut gespiegelt werden kann.

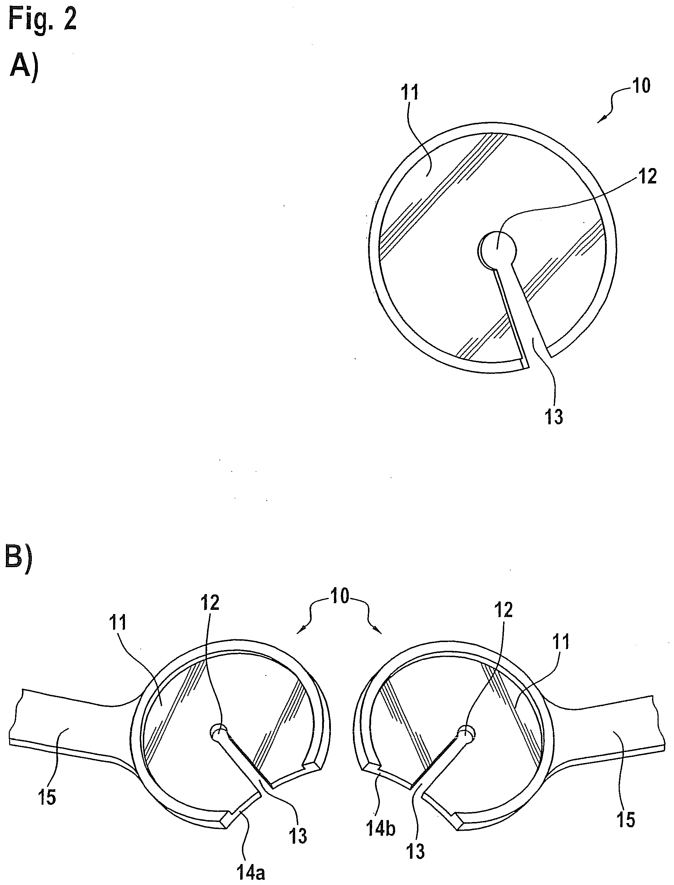

In Figur 2A ist ein erfindungsgemäßer Spiegel gezeigt, der wiederum ein Loch (12) in der Spiegelfläche (11) hat, wobei zwischen dem Außenrand der Spiegelfläche (11) und dem Loch (12) ein Spalt (13) verläuft. Dieser kann in vorteilhafter Weise dazu verwendet werden ein Dentalinstrument, beispielsweise ein zur Wurzelbehandlung verwendetes Instrument, das meist länglich ist, in das Loch seitlich einzuschieben, ohne dass es von der Spitze an durch das Loch eingeschoben werden muss. So ist es zum Beispiel auch möglich, den Spiegel während der Behandlung zu entfernen, ohne dass das Instrument aus der Behandlungsstelle herausgezogen werden muss.

Figur 2B zeigt zwei erfindungsgemäße Dentalspiegel (10) mit einer Spiegelfläche (11) und einem Griffstück (15) sowie einem Loch (12). Das Loch (12) ist wieder mit einer Spalte (13) zum Ein- und Ausführen eines Dentalinstruments versehen, wobei die Spalte (13) in einer konkaven Ausnehmung (14a, 14b) mündet. In den Figuren A und B sind die Spalten (13) und die konkaven Ausnehmungen (14a, 14b) seitenverkehrt angebracht. Somit kann das jeweilige passende Instrument ausgewählt werden je nachdem, ob der behandelnde Arzt oder die den Spiegel haltende Helferin diesen mit der rechten Hand oder mit der linken Hand hält und je nachdem, ob ein Zahn im rechten Kieferbereich oder im linken Kieferbereich bzw. im Oberkiefer oder Unterkiefer behandelt wird. Der Winkel in dem der Spalt (13) und/oder die Mitte der Ausnehmung (14a, 14b) zu der Längsachse des Griffstücks (15) versetzt ist, kann von 0° bis90° betragen, wobei ein Winkel um ca. 45° wie gezeigt bevorzugt ist.

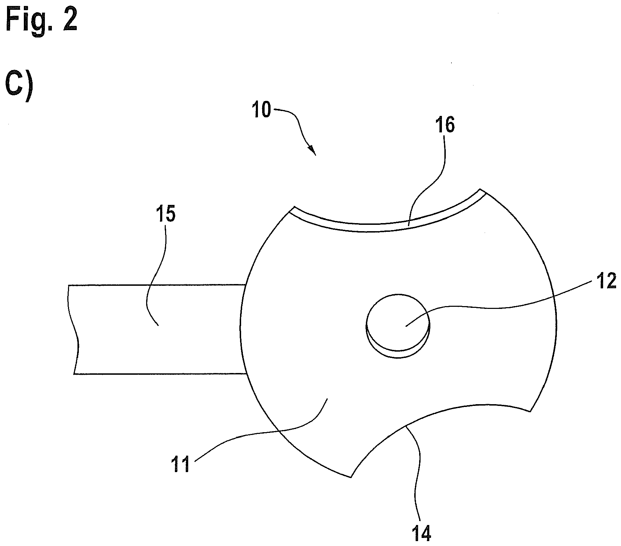

Figur 2C zeigt einen erfindungsgemäßen Spiegel, der wiederum eine Spiegelfläche (11) und ein Griffstück (15) aufweist, wobei in die Spiegelfläche wieder ein Loch (12) integriert ist. Der Spiegel (10) weist eine konkave Einbuchtung (14) auf. Diese kann in vorteilhafter Weise dazu verwendet werden, den Spiegel besser an den Zahn heranzubringen, da die sich normalerweise aus dem kreisförmigen Aufbau eines Dentalspiegels ergebende Ausbuchtung entfällt. Weiterhin ist eine zweite Ausbuchtung (16) gezeigt, die es ermöglicht, dass zum Beispiel ein Bohrkopf eines Winkelstücks einen möglichst großen Freiheitsgrad hat. In Figur 2B ist ein erfindungsgemäßer Spiegel gezeigt, der wiederum ein Loch (12) in der Spiegelfläche (11) hat, wobei zwischen dem Außenrand der Spiegelfläche (11) und dem Loch (12) ein Spalt (13) verläuft. Dieser kann in vorteilhafter Weise dazu verwendet werden ein Dentalinstrument, beispielsweise ein zur Wurzelbehandlung verwendetes Instrument, das meist länglich ist, in das Loch seitlich einzuschieben, ohne dass es von der Spitze an durch das Loch eingeschoben werden muss. So ist es zum Beispiel auch möglich, den Spiegel während der Behandlung zu entfernen, ohne dass das Instrument aus der Behandlungsstelle herausgezogen werden muss.

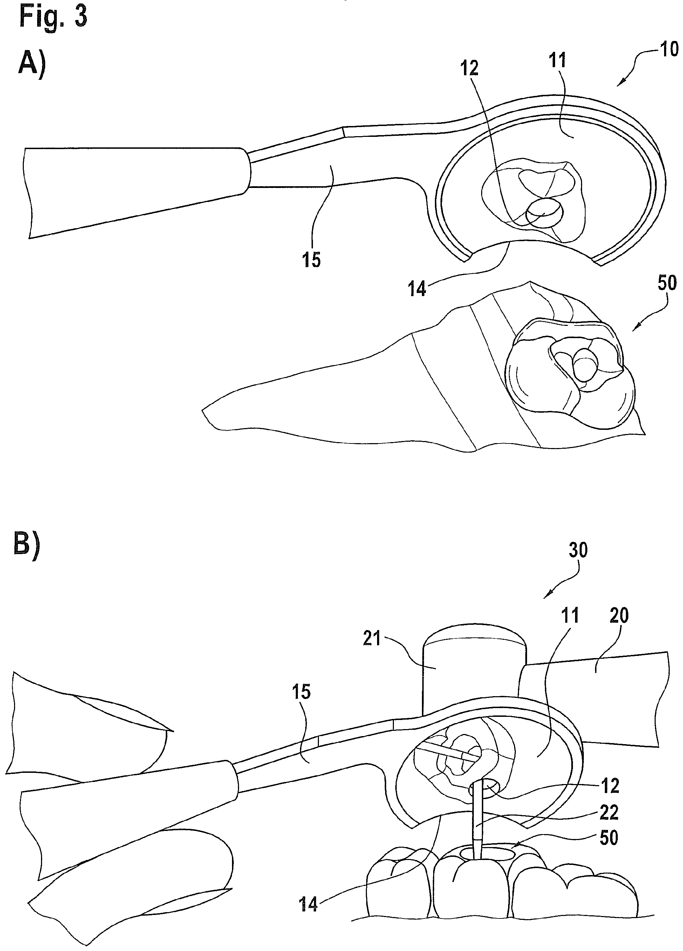

Figur 3A zeigt einen erfindungsgemäßen Dentalspiegel (10) mit einer konkaven Ausnehmung (14) und einem Griffstück (15) vor der Behandlung eines Zahns (50). Die Spiegelfläche (11) spiegelt die zu behandelnde Stelle eines Zahns (50, wobei dabei das Loch (12) durch seine Größe und Position den Sichtbereich nicht beeinträchtigt. Die konkave Ausnehmung (14) ermöglicht es in vorteilhafter Weise, dass der Spiegel (10) nahe an den Zahn (50) herangeführt werden kann.

Figur 3B zeigt eine erfindungsgemäße Vorrichtung (30), umfassend einen erfindungsgemäßen Dentalspiegel (10) und ein Dentalinstrument (20,21,22) bei der Behandlung eines Zahns (50). Der Dentalspiegel (10) hat wieder die konkave Ausnehmung (14) und das Griffstück (15). Das Dentalinstrument (30) weist ein Wurzelkanalinstrument (22), beispielsweise eine Nervennadel, eine Feile, eine Fräse, ein Bohrer oder ein Reamer und ein Winkelstück (21) und ein Wurzelkanalinstrument (22) auf. Während der Behandlung reicht das Wurzelkanalinstrument (22) durch das Loch (12) hindurch, so dass der Dentalspiegel (10) nicht im Weg ist. Gleichzeit kann der Zahnarzt aber über die Spiegelfläche (11) den Behandlungsverlauf genau beobachten und visuell kontrollieren.

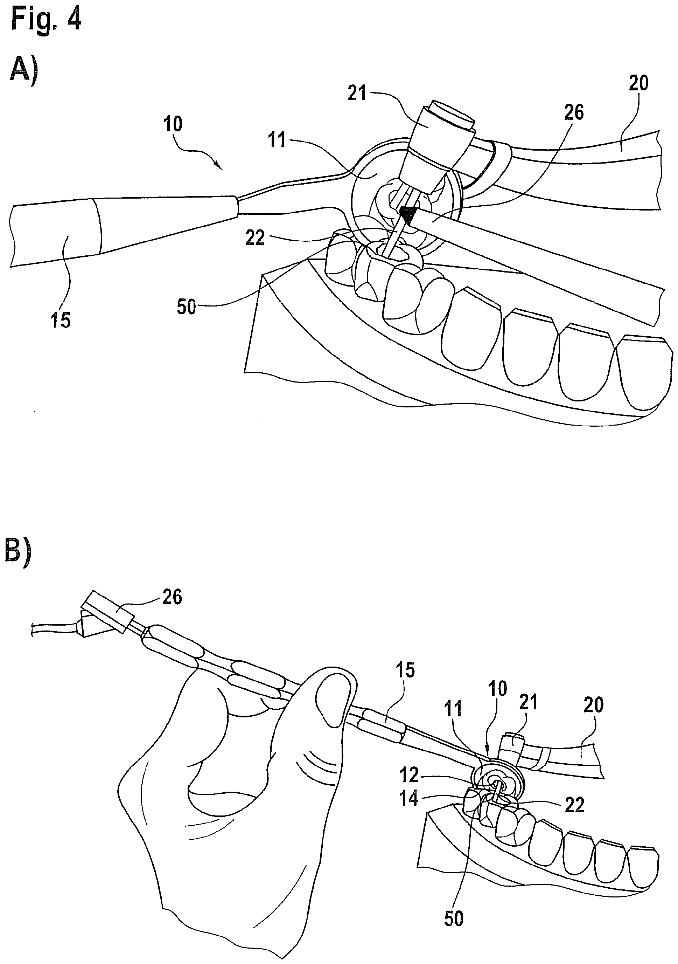

Figur 4A zeigt Herkömmliche Behandlung eines Zahns (50) mit einem Dentalspiegel (10) aus dem Stand der Technik und herkömmlicher elektronscher Wurzelkanaltiefenmessung (26). Das Dentalinstrument (20) weist ein Wurzelkanalinstrument (22) und ein Winkelstück (21) auf. Die Elektrode zur Wurzelkanaltiefenmessung (26) muss dabei an das Wurzelkanalinstrument (22) angedrückt werden. Die Elektrode (26) und der Spiegel (10) sind bei der Behandlung in diesen engen Platzverhältnissen im Weg und der Zahnarzt kann die zu behandelnde Stelle am Zahn (50) über den herkömmlichen Spiegel nur schwer und ungenau sehen.

Figur 4B zeigt eine erfindungsgemäße Behandlung und elektronscher Wurzelkanaltiefenmessung eines Zahns (50) mit einem erfindungsgemäßen Dentalspiegel (10) mit Spiegelfläche (11) und Loch (12) sowie konkaver Ausnehmung (14) und Griffstück (15). Zu sehen ist auch wieder ein Dentalinstrument (20) mit einem länglichen Wurzelkanalinstrument (22), beispielsweise eine Nervennadel, eine Feile, eine Fräse, ein Bohrer oder ein Reamer und einem Winkelstück (21), das das Wurzelkanalinstrument (22) hält. Das Wurzelkanalinstrument (22) reicht durch das Loch (12) des Dentalspiegels (10) hindurch und wird zur endodontalen Behandlung eines Zahns (50) verwendet.

Der Dentalspiegel (10) weist weiterhin am Griffstück (15) einen isolierten elektrischen Anschluss (26) auf, der über eine isolierte elektrische Leitung im Inneren des Griffstücks (15) das Loch (12) mit einem elektronischen Gerät zur Tiefenmessung verbunden werden kann. Die Wand des Lochs (12) oder ein Teil der Rückseite der Spiegelfläche (11) kann dabei aus einem elektrisch leitenden Material ausgebildet sein, das mit der isolierten elektrischen Leitung verbunden ist. Das Wurzelkanalinstrument (22) kann nun in vorteilhafter Weise diese elektrische Kontaktfläche am Loch (12) berühren, so dass eine elektronische Tiefenmessung während der Behandlung möglich ist, ohne dass eine Elektrode zur Wurzelkanaltiefenmessung an das Wurzelkanalinstrument herangehalten werden muss. Der Spiegel (10) kann durch die Größe des Lochs (12) leicht verdreht werden, ohne dass dies die Freigängigkeit des Wurzelkanalinstruments (22) stört. So kann der Zahnarzt den Spiegel in eine gewünschte Position drehen um die zu Behandelnde Stelle am Zahn (50) gut im Spiegelbild sehen zu können. Weder behindert der Spiegel (10) die Behandlung mit dem Wurzelkanalinstrument (22) noch Behindert das Dentalinstrument (20) dem Wurzelkanalinstrument (22) die Sicht über den Spiegel (10) auf den Zahn (50). Auch ist an der Behandlungsstelle keine störende Elektrode zur Wurzelkanaltiefenmessung nötig. Durch die konkave Ausnehmung (14) kann der Spiegel (10) besonders gut an den Zahn (50) herangeführt werden.

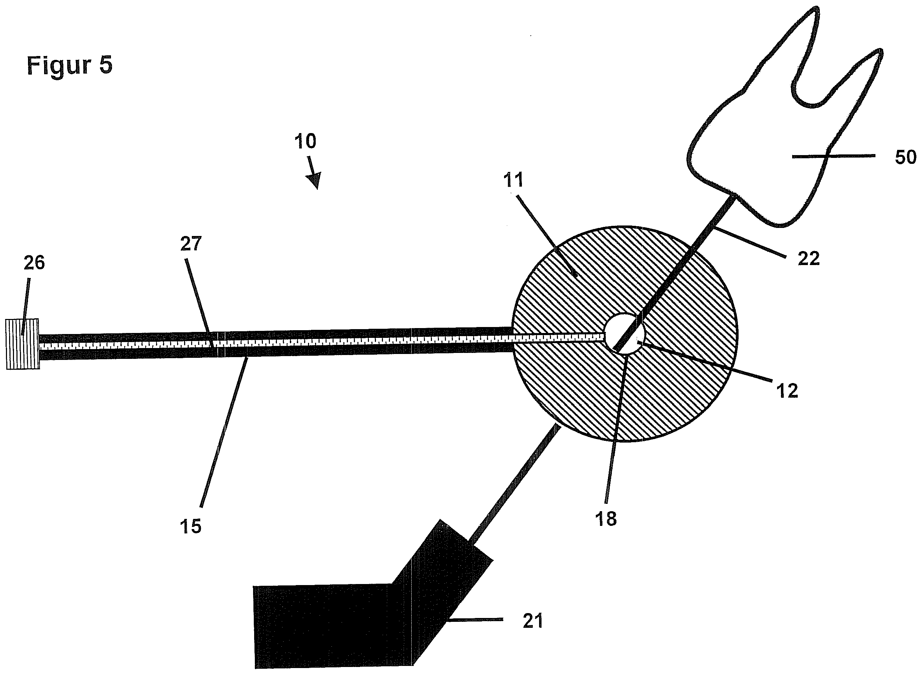

Figur 5 zeigt schematisch eine erfindungsgemäße Vorrichtung umfassend einen erfindungsgemäßen Dentalspiegel (10), ein längliches Wurzelkanalinstrument (22), beispielsweise eine Nervennadel, eine Feile, eine Fräse, ein Bohrer oder ein Reamer und ein Winkelstück (21), das das Wurzelkanalinstrument (22) hält. Der Dentalspiegel (10) weist wieder ein Griffstück (15) und eine Spiegelfläche (11) auf.

Das Wurzelkanalinstrument (22) reicht durch das Loch (12) des Dentalspiegels (10) hindurch und wird zur endodontalen Behandlung eines Zahns (50) verwendet. Natürlich kann der Dentalspiegel (10) weitere vorliegend beschriebene Merkmale aufweisen, beispielsweise die in den Figuren 2 und 3 gezeigten Spalten und Ausnehmungen.

Der Dentalspiegel (10) weist weiterhin am Griffstück (15) einen isolierten elektrischen Anschluss (26) auf, der über eine isolierte elektrische Leitung (27) das Loch (12) mit einem elektronischen Gerät zur Tiefenmessung verbunden werden kann. Die Wand (18) des Lochs (12) oder ein Teil der Rückseite der Spiegelfläche (11) kann dabei aus einem elektrisch leitenden Material ausgebildet sein, das mit der Leitung (27) verbunden ist. Das Wurzelkanalinstrument (22) kann nun in vorteilhafter Weise diese elektrische Kontaktfläche am Loch (12) berühren, so dass eine elektronische Tiefenmessung während der Behandlung möglich ist, ohne dass das Wurzelkanalinstrument mit einer störenden elektronischen Verbindung, beispielsweise einem Kabel, verbunden ist oder dieses an das Wurzelkanalinstrument (21) herangehalten werden muss. Diese Ausführungsform des Spiegels (10) der Vorrichtung kann natürlich auch bei einem einzelnen erfindungsgemäßen Spiegel vorgesehen sein. Auch kann vorgesehen sein, dass der Spiegel kein Griffstück aufweist, sondern mit dem Winkelstück verstellbar und verdrehbar verbunden ist.

## Patentansprüche

1. Dentalspiegel, aufweisend eine Spiegelfläche (11), wobei die Spiegelfläche ein Loch (12) zum Durchreichen eines Operationsinstruments (22) aufweist, wobei das Loch (12) über eine Spalte (13) mit der Spiegelflächenaußenkante verbunden ist und wobei die Spalte (13) in eine konkave Ausnehmung (14,14a,14b,16) mündet, **dadurch gekennzeichnet, dass** die Spiegelfläche (11) zwei konkave Ausnehmungen (14,14a,14b,16) aufweist und dass das Loch (12) auf der Rückseite der Spiegelfläche (11) abgeflachte oder abgerundete Kanten aufweist, wodurch eine Kippmöglichkeit des Spiegels entlang der Achse des durchreichenden Operationsinstruments (22) verbessert wird.

2. Dentalspiegel nach Anspruch 1, wobei das Loch (12) einen Durchmesser von mindestens 1 mm und höchstens 11 mm aufweist.

3. Dentalspiegel nach einem der vorhergehenden Ansprüche, wobei die Spiegelfläche (11) einen größten Durchmesser von höchstens 3,5 cm hat.

4. Dentalspiegel nach einem der vorhergehenden Ansprüche, wobei sich das Loch (12) etwa in der Mitte der Spiegelfläche (11) befindet.

5. Dentalspiegel nach einem der vorhergehenden Ansprüche, wobei die Spiegelfläche (11) rund ist.

6. Dentalspiegel nach einem der vorhergehenden Ansprüche, wobei der Dentalspiegel (10) ein Griffstück (15) aufweist.

7. Dentalspiegel nach einem der vorhergehenden Ansprüche, wobei die Spiegelfläche (11) eine vergrößernde Spiegelfläche ist.

8. Dentalspiegel nach einem der vorhergehenden Ansprüche, wobei der Dentalspiegel (10) am Griffstück (15) einen isolierten elektrischen Anschluss (26) aufweist, der über eine isolierte elektrische Leitung (27) mit einer aus einem elektrisch leitenden Material hergestellte Kontaktstelle (18) verbunden ist, wobei sich die Kontaktstelle an der Innenwandfläche des Lochs oder auf der Rückseite der Spiegelfläche (11) befindet.

9. Vorrichtung zur Behandlung von Zähnen, umfassend einen Dentalspiegel (10) nach einem der vorhergehenden Ansprüche und ein Winkelstück (21), welches mit dem durchreichenden Operationsinstrument verbunden ist, welches ein Dentalinstrument (22) ist.

10. Vorrichtung nach Anspruch 9 wobei die Vorrichtung zur endodontologischen Behandlung von Zähnen vorgesehen ist, wobei das Dentalinstrument ein Wurzelkanalinstrument (22) ist.

11. Vorrichtung nach Anspruch 10, wobei das Wurzelkanalinstrument (22) eine Nervennadel, eine Feile, ein Fräser, ein Bohrer oder ein Reamer ist.

## Claims

1. A dental mirror, having a mirror surface (11), wherein the mirror surface has a hole (12) for reaching through a surgical instrument (22), wherein the hole (12) is connected to the mirror surface outer edge via a gap (13) and wherein the gap (13) opens into a concave recess (14, 14a, 14b, 16), **characterised in that** the mirror surface (11) has two concave recesses (14, 14a, 14b, 16) and that the hole (12) has flattened or rounded edges at the back of the mirror surface (11), enhancing a tilting option of the mirror along the axis of the reaching-through surgical instrument (22).

2. The dental mirror according to claim 1, wherein the hole (12) has a diameter of at least 1 mm and of no more than 11 mm.

3. The dental mirror according to any one of the preceding claims, wherein the mirror surface (11) has a maximum diameter of no more than 3.5 cm.

4. The dental mirror according to any one of the preceding claims, wherein the hole (12) is located approximately at the centre of the mirror surface (11).

5. The dental mirror according to any one of the preceding claims, wherein the mirror surface (11) is round.

6. The dental mirror according to any one of the preceding claims, wherein the dental mirror (10) has a handle piece (15).

7. The dental mirror according to any one of the preceding claims, wherein the mirror surface (11) is a magnifying mirror surface.

8. The dental mirror according to any one of the preceding claims, wherein the dental mirror (10) has an insulated electrical connection (26) at the handle piece (15) which is connected via an insulated electrical cable (27) to a contact area (18) produced from an electrically conducting material, wherein the contact area is located on the inner wall surface of the hole or at the back of the mirror surface (11).

9. A device for treating teeth, comprising a dental mirror (10) according to any one of the preceding claims, and an angle piece (21) which is connected with the reaching-through surgical instrument, which is a dental instrument (22).

10. The device according to claim 9, wherein the device is provided for the endodontic treatment of teeth, wherein the dental instrument is a root canal instrument (22).

11. The device according to claim 10, wherein the root canal instrument (22) is a nerve broach, a file, a cutter, a drill or a reamer.

## Revendications

1. Miroir dentaire, présentant une surface de miroir (11), dans lequel la surface de miroir présente un trou (12) pour faire passer au travers un instrument opératoire (22), dans lequel le trou (12) est relié par le biais d'une fente (13) au bord extérieur de la surface de miroir et dans lequel la fente (13) débouche dans un évidement concave (14, 14a, 14b, 16), **caractérisé en ce que** la surface de miroir (11) présente deux évidements concaves (14, 14a, 14b, 16) et **en ce que** le trou (12) présente sur la face arrière de la surface de miroir (11) des bords aplatis ou arrondis, ce qui permet d'améliorer les possibilités de basculement du miroir le long de l'axe de l'instrument opératoire (22) traversant.

2. Miroir dentaire selon la revendication 1, dans lequel le trou (12) présente un diamètre au minimum de 1 mm et au maximum de 11 mm.

3. Miroir dentaire selon une des revendications précédentes, dans lequel la surface de miroir (11) a un diamètre le plus gros au maximum de 3,5 cm.

4. Miroir dentaire selon une des revendications précédentes, dans lequel le trou (12) se situe à peu près au centre de la surface de miroir (11).

5. Miroir dentaire selon une des revendications précédentes, dans lequel la surface de miroir (11) est ronde.

6. Miroir dentaire selon une des revendications précédentes, dans lequel le miroir dentaire (10) présente une poignée (15).

7. Miroir dentaire selon une des revendications précédentes, dans lequel la surface de miroir (11) est une surface de miroir grossissante.

8. Miroir dentaire selon une des revendications précédentes, dans lequel le miroir dentaire (10) présente sur la poignée (15) un raccordement électriquement isolé (26) qui est relié par le biais d'une ligne électriquement isolée (27) à une pièce de contact (18) réalisée en un matériau électriquement conducteur, la pièce de contact se situant sur la surface de la paroi intérieure du trou ou sur la face arrière de la surface de miroir (11).

9. Dispositif destiné à traiter des dents, comprenant un miroir dentaire (10) selon une des revendications précédentes et un coude (21) qui est relié à l'instrument opératoire traversant qui est un instrument dentaire (22).

10. Dispositif selon la revendication 9, dans lequel le dispositif est prévu pour le traitement endodontique de dents, dans lequel l'instrument dentaire est un instrument de canal radiculaire (22).

11. Dispositif selon la revendication 10, dans lequel l'instrument de canal radiculaire (22) est une aiguille nerveuse, une lime, une fraise, une perceuse ou un reamer.
